# EUROPEAN PATENT APPLICATION

(11) **EP 4 130 204 A1**
(43) Date of publication of application: **08.02.2023**
(21) Application number: 21776243.4
(22) Date of filing: 22.03.2021
(51) Int. Cl.: C10K 1/02, C10K 1/08, C10J 3/00

(54) **PYROLYSIS GAS PURIFICATION/COOLING DEVICE, PYROLYSIS GAS PURIFICATION/COOLING METHOD, ORGANIC SUBSTANCE PRODUCTION DEVICE, AND METHOD FOR PRODUCING ORGANIC SUBSTANCE**

(30) Priority: 24.03.2020 JP 2020052674; 31.03.2020 JP 2020064633
(71) Applicant: SEKISUI CHEMICAL CO., LTD., Osaka-shi Osaka 530-8565 (JP)
(72) Inventor: HAMACHI, Kokoro, Tokyo 105-8566 (JP)
(74) Representative: Ter Meer Steinmeister & Partner
(86) International application number: PCT/JP2021/011804
(87) International publication number: WO 2021/193573

(57) **Abstract**

Provided are a method for producing an organic substance and a device for producing an organic substance that are capable of efficiently cooling a synthesis gas and of converting the synthesis gas to an organic substance at a high conversion efficiency using a microbial catalyst. A thermally decomposed gas purification/cooling device including a gasification furnace 10 that gasifies waste to generate a thermally decomposed gas, a cyclone 11 through which the thermally decomposed gas discharged from the gasification furnace 10 is passed to recover a dust component in the thermally decomposed gas, and a heat exchanger 20 through which the thermally decomposed gas that has passed through the cyclone 11 is passed to be cooled.

## Description

### Technical Field

The present invention relates to a thermally decomposed gas purification/cooling device and a thermally decomposed gas purification/cooling method of a thermally decomposed gas derived from waste and a device for producing an organic substance and a method for producing an organic substance that produce an organic substance using a synthesis gas derived from waste as a raw material.

### Background Art

Techniques that thermally decompose a variety of wastes such as industrial waste and general waste to generate a thermally decomposed gas in a gasification furnace and then reform the thermally decomposed gas generated to obtain a synthesis gas in a reforming furnace are broadly known. The obtained synthesis gas is combusted as it is and used for power generation or the like or is used for power generation or the like after heat is recovered with a boiler or the like as necessary.

In addition, recently, attempts have been underway to use a synthesis gas as a chemical synthesis raw material, and, for example, attempts have been underway to convert a synthesis gas to an organic substance such as ethanol using a microbial catalyst (for example, refer to PTL 1).

Thermally decomposed gases derived from waste contain impurities such as tar, char and the like in large quantities, and it is difficult to use thermally decomposed gases as they are for power generation and chemical synthesis. In addition, when substances containing components other than carbon in large quantities are gasified, thermally decomposed gases derived from waste contain phase-transitable impurities capable of phase transition between gaseous and solid phases, including a sublimable substance such as naphthalene, 1-naphthol or 2-naphthol. Thus, it is ordinary to use thermally decomposed gases derived from waste after the gases are purified to remove such impurities.

### Citation List

### Patent Literature

PTL1: International Publication No. WO 2015/037710

### Summary of Invention

### Technical Problem

Thermally decomposed gases derived from waste are hot when generated, but there is a case where thermally decomposed gases are required to be cooled to a low temperature when used. For example, in a case where a thermally decomposed gas is converted to an organic substance such as ethanol with a microbial catalyst, the thermally decomposed gas is required to be cooled to 40°C or lower. Therefore, it is necessary to cool thermally decomposed gases derived from waste while purifying the gases. However, when a thermally decomposed gas derived from waste is cooled, there is a case where a phase-transitable impurity in the gas is precipitated in a cooler, a filter or the like and dust components such as tar and char are adsorbed to and enlarged on the surface thereof, which causes a decrease in cooling efficiency or blockage of a gas flow path. In order to avoid these phenomena, there is a need to clean phase-transitable impurities and dust components frequently, which creates a problem in that maintenance becomes complicated and the operation cost increases.

Therefore, an objective of the present invention is to provide a thermally decomposed gas purification/cooling device and a thermally decomposed gas purification/cooling method that are capable of efficiently removing an impurity from thermally decomposed gases derived from waste by preventing a decrease in cooling efficiency, blockage of a gas flow path or the like and of suitably cooling and purifying thermally decomposed gases and a device for producing an organic substance and a method for producing an organic substance in which an organic substance is produced using a synthesis gas derived from water as a raw material.

### Solution to Problem

As a result of intensive studies, the present inventors found that the above-described problem can be solved by disposing a cyclone in the post-stage of a gasification furnace and passing a thermally decomposed gas discharged from the gasification furnace through the cyclone and completed a first form of the present invention to be described below.

That is, the first form of the present invention provides [1] to [28] below.
[1] A thermally decomposed gas purification/cooling device including a gasification furnace that gasifies waste to generate a thermally decomposed gas, a cyclone through which the thermally decomposed gas discharged from the gasification furnace is passed to recover a dust component in the thermally decomposed gas, and a heat exchanger through which the thermally decomposed gas that has passed through the cyclone is passed to be cooled.
[2] The thermally decomposed gas purification/cooling device according to [1], in which a temperature of the thermally decomposed gas to be supplied to the cyclone is 500°C or higher and 1,100°C or lower.
[3] The thermally decomposed gas purification/cooling device according to [1] or [2], in which the thermally decomposed gas is cooled to a temperature of 30°C or higher and 300°C or lower in the heat exchanger.
[4] The thermally decomposed gas purification/cooling device according to any of [1] to [3], further including a reforming furnace that is disposed in a post-stage of the cyclone and reforms the thermally decomposed gas discharged from the gasification furnace.
[5] The thermally decomposed gas purification/cooling device according to any of [1] to [3], further including a reforming furnace that is disposed in a pre-stage of the cyclone and reforms the thermally decomposed gas discharged from the gasification furnace.
[6] The thermally decomposed gas purification/cooling device according to any of [1] to [5], in which the dust component recovered in the cyclone is supplied to the gasification furnace.
[7] The thermally decomposed gas purification/cooling device according to any of [1] to [6], further including a gas cooling tower which is disposed in a post-stage of the heat exchanger and through which the thermally decomposed gas cooled in the heat exchanger is passed through to be cooled by water spray.
[8] The thermally decomposed gas purification/cooling device according to any of [1] to [7], further including a filtration-type dust collector which is disposed in a post-stage of the heat exchanger and through which the thermally decomposed gas cooled in the heat exchanger is passed.
[9] The thermally decomposed gas purification/cooling device according to any of [1] to [8], further including a scrubber which is disposed in a post-stage of the heat exchanger and through which the thermally decomposed gas cooled in the heat exchanger is passed.
[10] The thermally decomposed gas purification/cooling device according to any of [1] to [9], further including a filtration-type dust collector and a scrubber, in which the filtration-type dust collector and the scrubber are disposed in parallel in a post-stage of the heat exchanger.
[11] The thermally decomposed gas purification/cooling device according to [10], further including a differential pressure-measuring device that measures a differential pressure between a pre-stage and a post-stage of the filtration-type dust collector.
[12] The thermally decomposed gas purification/cooling device according to [10] or [11], further including a concentration-measuring device that measures a concentration of at least any selected from a phase-transitable impurity and a solid impurity in the thermally decomposed gas discharged from the gasification furnace.
[13] The thermally decomposed gas purification/cooling device according to any of [10] to [12], further including a flow path-switching portion that selectively switches a supply destination to the filtration-type dust collector or the scrubber through which the thermally decomposed gas is passed.
[14] A device for producing an organic substance including an organic substance generation portion that generates an organic substance by bringing a synthesis gas obtained by treating a thermally decomposed gas with the thermally decomposed gas purification/cooling device according to any of [1] to [13] into contact with a microbial catalyst.
[15] A thermally decomposed gas purification/cooling method including a step of gasifying waste with a gasification furnace to generate a thermally decomposed gas, a step of passing the thermally decomposed gas discharged from the gasification furnace through a cyclone to recover a dust component in the thermally decomposed gas, and a step of passing the thermally decomposed gas that has passed through the cyclone through a heat exchanger to cool the thermally decomposed gas.
[16] The thermally decomposed gas purification/cooling method according to [15], in which a temperature of the thermally decomposed gas to be supplied to the cyclone is 500°C or higher and 1,100°C or lower.
[17] The thermally decomposed gas purification/cooling method according to [15] or [16], in which the thermally decomposed gas is cooled to a temperature of 30°C or higher and 300°C or lower in the heat exchanger.
[18] The thermally decomposed gas purification/cooling method according to any of [15] to [17], further including, in a post-stage where the thermally decomposed gas has passed through the cyclone, a step of passing the thermally decomposed gas discharged from the gasification furnace through a reforming furnace to reform the thermally decomposed gas.
[19] The thermally decomposed gas purification/cooling method according to any of [15] to [18], further including, in a pre-stage where the thermally decomposed gas is to be passed through the cyclone, a step of passing the thermally decomposed gas discharged from the gasification furnace through a reforming furnace to reform the thermally decomposed gas.
[20] The thermally decomposed gas purification/cooling method according to any of [15] to [19], in which the dust component recovered in the cyclone is supplied to the gasification furnace.
[21] The thermally decomposed gas purification/cooling method according to any of [15] to [20], further including a step of passing the thermally decomposed gas cooled in the heat exchanger through a gas cooling tower to cool the thermally decomposed gas with water sprayed in the gas cooling tower.
[22] The thermally decomposed gas purification/cooling method according to any of [15] to [21], further including a step of passing the thermally decomposed gas cooled in the heat exchanger through a filtration-type dust collector.
[23] The thermally decomposed gas purification/cooling method according to any of [15] to [22], further including a step of passing the thermally decomposed gas cooled in the heat exchanger through a scrubber.
[24] The thermally decomposed gas purification/cooling method according to any of [15] to [23], further including a step of passing the thermally decomposed gas through any of a filtration-type dust collector and a scrubber that are disposed in parallel in a post-stage of the heat exchanger.
[25] The thermally decomposed gas purification/cooling method according to [24], further including a step of measuring a differential pressure between a pre-stage and a post-stage of the filtration-type dust collector with a differential pressure-measuring device.
[26] The thermally decomposed gas purification/cooling method according to [24] or [25], further including a step of measuring a concentration of at least any selected from a phase-transitable impurity and a solid impurity in the thermally decomposed gas discharged from the gasification furnace with a concentration-measuring device.
[27] The thermally decomposed gas purification/cooling method according to [25] or [26], further including a step of selectively switching supply of the thermally decomposed gas to the filtration-type dust collector or the scrubber with a flow path-switching portion depending on a measurement result of at least any of the differential pressure-measuring device and the concentration-measuring device.
[28] A method for producing an organic substance including a step of bringing a synthesis gas obtained by treating a thermally decomposed gas with the thermally decomposed gas purification/cooling method according to any of [15] to [27] into contact with a microbial catalyst to generate an organic substance.

The present invention also provides the following second form. In the second form, it is possible to obtain a synthesis gas having a high content rate of at least any of hydrogen and carbon monoxide and appropriately cooled with a heat exchanger.

That is, the second form of the present invention provides [29] to [48] below.
[29] A thermally decomposed gas purification/cooling device including a gasification furnace that gasifies waste to generate a thermally decomposed gas, a reforming furnace that reforms the thermally decomposed gas discharged from the gasification furnace, and a heat exchanger through which the thermally decomposed gas that has passed through the reforming furnace is passed to be cooled.
[30] The thermally decomposed gas purification/cooling device according to [29], in which the thermally decomposed gas is cooled to a temperature of 30°C or higher and 300°C or lower in the heat exchanger.
[31] The thermally decomposed gas purification/cooling device according to [29] or [30], further including a gas cooling tower which is disposed in a post-stage of the heat exchanger and through which the thermally decomposed gas cooled in the heat exchanger is passed through to be cooled by water spray.
[32] The thermally decomposed gas purification/cooling device according to any of [29] to [31], further including a filtration-type dust collector which is disposed in a post-stage of the heat exchanger and through which the thermally decomposed gas cooled in the heat exchanger is passed.
[33] The thermally decomposed gas purification/cooling device according to any of [29] to [32], further including a scrubber which is disposed in a post-stage of the heat exchanger and through which the thermally decomposed gas cooled in the heat exchanger is passed.
[34] The thermally decomposed gas purification/cooling device according to any of [29] to [33], further including a filtration-type dust collector and a scrubber, in which the filtration-type dust collector and the scrubber are disposed in parallel in a post-stage of the heat exchanger.
[35] The thermally decomposed gas purification/cooling device according to [34], further including a differential pressure-measuring device that measures a differential pressure between a pre-stage and a post-stage of the filtration-type dust collector.
[36] The thermally decomposed gas purification/cooling device according to [34] or [35], further including a concentration-measuring device that measures a concentration of at least any selected from a phase-transitable impurity and a solid impurity in the thermally decomposed gas discharged from the gasification furnace.
[37] The thermally decomposed gas purification/cooling device according to any of [34] to [36], further including a flow path-switching portion that selectively switches a supply destination to the filtration-type dust collector or the scrubber through which the thermally decomposed gas is passed.
[38] A device for producing an organic substance including an organic substance generation portion that generates an organic substance by bringing a synthesis gas obtained by treating a thermally decomposed gas with the thermally decomposed gas purification/cooling device according to any of [29] to [37] into contact with a microbial catalyst.
[39] A thermally decomposed gas purification/cooling method including a step of gasifying waste with a gasification furnace to generate a thermally decomposed gas, a step of passing the thermally decomposed gas discharged from the gasification furnace through a reforming furnace to reform the thermally decomposed gas, and a step of passing the thermally decomposed gas that has passed through the reforming furnace through a heat exchanger to cool the thermally decomposed gas.
[40] The thermally decomposed gas purification/cooling method according to [39], in which the thermally decomposed gas is cooled to a temperature of 30°C or higher and 300°C or lower in the heat exchanger.
[41] The thermally decomposed gas purification/cooling method according to [39] or [40], further including a step of passing the thermally decomposed gas cooled in the heat exchanger through a gas cooling tower to cool the thermally decomposed gas with water sprayed in the gas cooling tower.
[42] The thermally decomposed gas purification/cooling method according to any of [39] to [41], further including a step of passing the thermally decomposed gas cooled in the heat exchanger through a filtration-type dust collector.
[43] The thermally decomposed gas purification/cooling method according to any of [39] to [42], further including a step of passing the thermally decomposed gas cooled in the heat exchanger through a scrubber.
[44] The thermally decomposed gas purification/cooling method according to any of [39] to [43], further including a step of passing the thermally decomposed gas through any of a filtration-type dust collector and a scrubber that are disposed in parallel in a post-stage of the heat exchanger.
[45] The thermally decomposed gas purification/cooling method according to [44], further including a step of measuring a differential pressure between a pre-stage and a post-stage of the filtration-type dust collector with a differential pressure-measuring device.
[46] The thermally decomposed gas purification/cooling method according to [44] or [45], further including a step of measuring a concentration of at least any selected from a phase-transitable impurity and a solid impurity in the thermally decomposed gas discharged from the gasification furnace with a concentration-measuring device.
[47] The thermally decomposed gas purification/cooling method according to [45] or [46], further including a step of selectively switching supply of the thermally decomposed gas to the filtration-type dust collector or the scrubber with a flow path-switching portion depending on a measurement result of at least any of the differential pressure-measuring device and the concentration-measuring device.
[48] A method for producing an organic substance including a step of bringing a synthesis gas obtained by treating a thermally decomposed gas with the thermally decomposed gas purification/cooling method according to any of [39] to [47] into contact with a microbial catalyst to generate an organic substance.

### Advantageous Effects of Invention

According to the present invention, it becomes possible to provide a thermally decomposed gas purification/cooling device and a thermally decomposed gas purification/cooling method that are capable of efficiently removing an impurity from thermally decomposed gases derived from waste by preventing a decrease in cooling efficiency, blockage of a gas flow path or the like and of suitably cooling and purifying thermally decomposed gases and a device for producing an organic substance and a method for producing an organic substance in which an organic substance is produced using a synthesis gas derived from waste as a raw material.

### Brief Description of the Drawings

[Fig. 1] Fig. 1 is a schematic view showing the overall configuration of a device for producing an organic substance according to a first embodiment of the present invention.
[Fig. 2] Fig. 2 is a schematic view showing the configuration of a thermally decomposed gas purification/cooling device according to the first embodiment of the present invention.
[Fig. 3] Fig. 3 is a schematic view showing a first modification example of the configuration of the thermally decomposed gas purification/cooling device according to the first embodiment of the present invention.
[Fig. 4] Fig. 4 is a schematic view showing a second modification example of the configuration of the thermally decomposed gas purification/cooling device according to the first embodiment of the present invention.
[Fig. 5] Fig. 5 is a schematic view showing a third modification example of the configuration of the thermally decomposed gas purification/cooling device according to the first embodiment of the present invention.
[Fig. 6] Fig. 6 is a schematic view showing a fourth modification example of the configuration of the thermally decomposed gas purification/cooling device according to the first embodiment of the present invention.
[Fig. 7] Fig. 7 is a schematic view showing the overall configuration of a device for producing an organic substance according to a second embodiment of the present invention.
[Fig. 8] Fig. 8 is a schematic view showing the configuration of a thermally decomposed gas purification/cooling device according to a third embodiment of the present invention.
[Fig. 9] Fig. 9 is a schematic view showing the configuration of a first modification example of the thermally decomposed gas purification/cooling device according to the third embodiment of the present invention.
[Fig. 10] Fig. 10 is a schematic view showing the configuration of a second modification example of the thermally decomposed gas purification/cooling device according to the third embodiment of the present invention.
[Fig. 11] Fig. 11 is a schematic view showing the configuration of a third modification example of the thermally decomposed gas purification/cooling device according to the third embodiment of the present invention.
[Fig. 12] Fig. 12 is a schematic view showing the overall configuration of a device for producing an organic substance according to a fourth embodiment of the present invention.

### Description of Embodiments

Hereinafter, embodiments of the present invention will be described with reference to drawings. In the following description of the drawings, the same or similar portions will be indicated by the same or similar reference signs.

### (First embodiment)

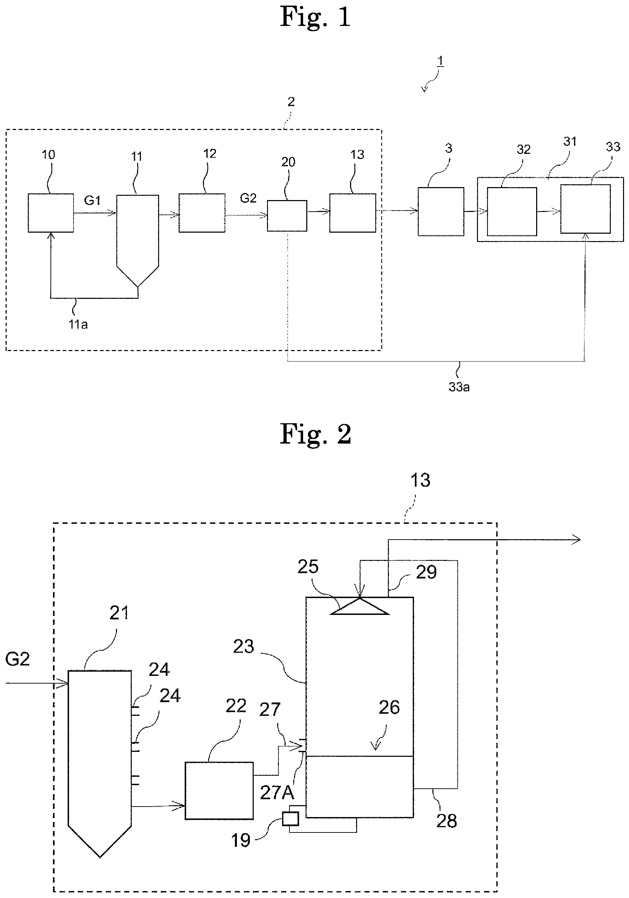

As shown in Fig. 1, a device for producing an organic substance 1 according to a first embodiment of the present invention includes a thermally decomposed gas purification/cooling device 2. Hereinafter, the device for producing an organic substance 1 and a method for producing an organic substance according to the first embodiment of the present invention will be described in detail with reference to the embodiment.

The device for producing an organic substance 1 includes the thermally decomposed gas purification/cooling device 2 that gasifies waste to generate a thermally decomposed gas G1 and carries out a treatment including at least a purification treatment and a cooling treatment on the thermally decomposed gas G1 and an organic substance generation portion 3 that brings a synthesis gas G2 obtained by treating the thermally decomposed gas G1 with the thermally decomposed gas purification/cooling device 2 into contact with a microbial catalyst to generate an organic substance.

### (Thermally decomposed gas purification/cooling device)

As shown in Fig. 1, the thermally decomposed gas purification/cooling device 2 in the first embodiment includes at least a gasification furnace 10, a cyclone 11 that is disposed in the post-stage of the gasification furnace 10 and a heat exchanger 20 that is disposed in the post-stage of the cyclone 11. The thermally decomposed gas purification/cooling device 2 further includes a reforming furnace 12 in the post-stage of the cyclone 11 and in the pre-stage of the heat exchanger 20. The thermally decomposed gas purification/cooling device 2 further includes one or more treatment devices (hereinafter, these devices will be collectively referred to as the "post-stage treatment device 13" in some cases) in the post-stage of the heat exchanger 20.

"The post-stage" in the present specification means the post-stage along the gas supply flow of the thermally decomposed gas G1. In addition, "the pre-stage" means the pre-stage along the supply flow of the thermally decomposed gas G1. The supply flow of the thermally decomposed gas G1 means the flow of the thermally decomposed gas G1 while the thermally decomposed gas G1 is discharged from the gasification furnace 10 and the synthesis gas G2 reformed in the reforming furnace 12 is introduced into the organic substance generation portion 3.

### <Gasification furnace>

The gasification furnace 10 is a device that generates the thermally decomposed gas G1 derived from waste by the combustion, thermal decomposition or the like of waste. The waste that is gasified in the gasification furnace 10 may be industrial waste such as industrial solid waste or may be general waste such as municipal solid waste (MSW), and examples thereof include combustible substances such as plastic waste, raw garbage, discarded tires, biomass waste, food waste, building materials, wood, wooden chips, fibers and paper. Among these, municipal solid waste (MSW) is preferable.

The gasification furnace 10 is not particularly limited, and examples thereof include a kiln gasification furnace, a fixed-bed gasification furnace, a fluidized-bed gasification furnace, a shaft furnace, a thermoselect furnace, a plasma gasification furnace and the like. Into the gasification furnace 10, not only waste but also oxygen or air and, furthermore, water vapor, if necessary, are injected. In the gasification furnace 10, the waste is heated at, for example, 500°C to 1,100°C, preferably, 500°C to 700°C and thereby thermally decomposed and partially oxidized as appropriate to be gasified. The thermally decomposed gas G1 contains not only carbon monoxide and hydrogen but also tar, char and the like. The thermally decomposed gas G1 is supplied to the thermally decomposed gas purification/cooling device 2. A solid matter or the like that is generated as an impurity in the gasification furnace 10 is recovered as appropriate.

### <Cyclone>

The cyclone 11 is a device in which the thermally decomposed gas G1 obtained in the gasification furnace 10 is introduced, the thermally decomposed gas G1 that is passing through the cyclone 11 is swirled to generate a centrifugal force, and a dust component, which is a solid that is contained in the thermally decomposed gas G1, is separated and removed by the centrifugal force and recovered. In the present specification, the "dust component" refers to solid components such as tar and char that are contained in the thermally decomposed gas G1. The dust component is separated and removed from the thermally decomposed gas G1 with the cyclone 11, whereby the content of the dust component in the thermally decomposed gas G1 can be reduced, and it is possible to prevent a decrease in cooling efficiency or blockage of a gas flow path in the post-stage, which arises from the dust component. The thermally decomposed gas G1 from which the dust component has been separated and removed with the cyclone 11 is supplied to the reforming furnace 12.

In the present specification, "remove" means that the concentration of a target substance to be removed in the gas is reduced by removing at least a part of the target substance from the synthesis gas and is not limited to the complete removing of the target substance to be removed.

The temperature of the thermally decomposed gas G1 to be supplied to the cyclone 11 is not particularly limited as long as the temperature is within the heatproof temperature range of the cyclone 11, but is, for example, 500°C or higher and 1,100°C or lower, preferably 500°C or higher and 900°C or lower and more preferably 500°C or higher and 700°C or lower. When the temperature of the thermally decomposed gas G1 to be supplied to the cyclone 11 is set within the above-described range, it is possible to suitably separate and remove the dust component.

The dust component separated, removed and recovered with the cyclone 11 is preferably reused and more preferably supplied to the gasification furnace 10 through a dust component supply path 11a provided in the cyclone 11. Since a main component of the dust component separated and removed with the cyclone 11 is a carbon component, the resupply of the dust component to the gasification furnace 10 makes it possible to adjust the content rate of carbon monoxide in the synthesis gas G2 to be described below.

### <Reforming furnace>

In the reforming furnace 12, the thermally decomposed gas G1 obtained in the gasification furnace 10 is reformed, the content rate of at least any of hydrogen and carbon monoxide in the thermally decomposed gas G1 increases, and the thermally decomposed gas is discharged as the synthesis gas G2. In the reforming furnace 12, for example, tar, char and the like that are contained in the thermally decomposed gas G1 are reformed into hydrogen, carbon monoxide and the like.

The temperature of the synthesis gas G2 in the reforming furnace 12 is not particularly limited, but is, for example, 900°C or higher, preferably 900°C or higher and 1,300°C or lower and more preferably 1,000°C or higher and 1,200°C or lower. When the temperature in the reforming furnace 12 is set within the above-described range, it becomes easy to obtain the synthesis gas G2 in which the content rates of carbon monoxide and hydrogen are high.

The temperature of the synthesis gas G2 that is discharged from the reforming furnace 12 is the same as the temperature of the synthesis gas G2 and is, for example, 900°C or higher, preferably 900°C or higher and 1,300°C or lower and more preferably 1,000°C or higher and 1,200°C or lower.

The synthesis gas G2 that is discharged from the reforming furnace 12 contains carbon monoxide and hydrogen. In addition, the synthesis gas G2 contains, for example, 0.1 vol% or more and 80 vol% or less of carbon monoxide and 0.1 vol% or more and 80 vol% or less of hydrogen.

The carbon monoxide concentration in the synthesis gas G2 is preferably 10 vol% or more and 70 vol% or less and more preferably 20 vol% or more and 55 vol% or less. In addition, the hydrogen concentration in the synthesis gas G2 is preferably 10 vol% or more and 70 vol% or less and more preferably 20 vol% or more and 55 vol% or less.

The synthesis gas G2 may contain, in addition to hydrogen and carbon monoxide, carbon dioxide, nitrogen, oxygen and the like. The carbon dioxide concentration in the synthesis gas G2 is not particularly limited, but is preferably 0.1 vol% or more and 40 vol% or less and more preferably 0.3 vol% or more and 30 vol% or less. In the case of generating ethanol using a microbial catalyst, it is particularly preferable to decrease the carbon dioxide concentration, and, from such a viewpoint, the carbon dioxide concentration is more preferably 0.5 vol% or more and 25 vol% or less.

The nitrogen concentration in the synthesis gas G2 is ordinarily 40 vol% or less and preferably 1 vol% or more and 20 vol% or less.

In addition, the oxygen concentration in the synthesis gas G2 is ordinarily 5 vol% or less and preferably 1 vol% or less. In addition, the oxygen concentration is preferably as low as possible as long as the oxygen concentration is 0 vol% or more. However, ordinarily, oxygen is inevitably contained in many cases, and the oxygen concentration is practically 0.01 vol% or more.

The concentrations of carbon monoxide, carbon dioxide, hydrogen, nitrogen and oxygen in the synthesis gas G2 can be set within predetermined ranges by appropriately changing combustion conditions such as the kind of the waste, the temperatures of the gasification furnace 10 and the reforming furnace 12 and the oxygen concentration of a supply gas that is supplied to the gasification furnace 10. For example, in a case where there is a desire to change the carbon monoxide or hydrogen concentration, the waste is changed to waste in which the rate of hydrocarbon (carbon and hydrogen) is high such as plastic waste, and, in a case where there is a desire to decrease the nitrogen concentration, a gas having a high oxygen concentration in the gasification furnace 10 is supplied.

Furthermore, in the synthesis gas G2, the concentration of each component such as carbon monoxide, carbon dioxide, hydrogen and nitrogen may be appropriately adjusted. The concentration is preferably adjusted by adding at least one of these components to the synthesis gas G2.

The volume percentage of each substance in the synthesis gas G2 means the volume percentage of each substance in the synthesis gas G2 that is discharged from the reforming furnace 12.

### <Heat exchanger>

The synthesis gas G2 discharged from the reforming furnace 12 passes through the heat exchanger 20. The heat exchanger 20 is a device that cools the synthesis gas G2 using a heat medium. The heat exchanger 20 cools the synthesis gas G2 by transferring the heat energy of the synthesis gas G2 to the heat medium.

As the heat exchanger 20, a boiler is preferably used. The boiler is a device in which water is communicated as a heat medium, and the communicated water is heated by the heat energy of the synthesis gas G2 and turned into vapor. When the boiler is used as the heat exchanger 20, it becomes possible to easily heat other devices with vapor generated from the boiler, and the heat energy of the synthesis gas G2 can be easily reused.

Here, the heat exchanger 20 to be used can be a device other than the boiler. The heat exchanger 20 other than the boiler may have any configuration as long as the heat energy is transferred to the heat medium from the synthesis gas G2, but a partition method in which the synthesis gas G2 and the heat medium do not come into direct contact with each other is preferable. The heat medium may be any of gas or liquid and may be a heat medium accompanying a phase change between gas and liquid. In addition, the heat energy from the synthesis gas G2 may be transferred to the heat medium in a state of having passed through a path with any shape such as a tubular shape or a plate shape. When the boiler is used as the heat exchanger 20, it is difficult to cool the synthesis gas G2 to a low temperature of, for example, 100°C or lower, but the use of the heat exchanger 20 other than boilers makes it possible to cool the synthesis gas G2 even to a low temperature of 100°C or lower. In addition, as the heat exchanger 20, two or more heat exchangers may be combined together, and, for example, a boiler and a heat exchanger other than boilers may be combined together.

As described above, the heat exchanger 20 cools the synthesis gas G2 supplied at a high temperature of, for example, 900°C or higher, for example, cools to a temperature of 30°C or higher and 300°C or lower, preferably 40°C or higher and 240°C or lower. Ordinarily, when the synthesis gas G2 containing a phase-transitable impurity and the dust component is cooled to 240°C or lower, the phase-transitable impurity such as naphthalene is solidified and precipitated, and the dust component is adsorbed to and enlarged on the surface of the precipitated phase-transitable impurity, which causes blockage of a gas flow path. However, the synthesis gas G2 to be supplied to the heat exchanger 20 has passed through the cyclone 11, whereby the dust component has been separated and removed in the cyclone 11. Therefore, even when the synthesis gas G2 is cooled to 240°C or lower, it is possible to suppress enlargement by the dust component since the dust component in the synthesis gas G2 has been removed. Therefore, it is possible to sufficiently cool the synthesis gas G2 in the heat exchanger 20 and to reduce a load of cooling in the post-stage treatment device. In addition, the synthesis gas G2 cooled to 100°C or lower also makes it possible not to provide a part of cooling devices in the post-stage (for example, a cooling tower to be described below). In addition, for example, when the synthesis gas G2 is cooled to approximately 40°C in the heat exchanger 20, it is possible to supply the synthesis gas G2 at an appropriate temperature for a microbial catalyst to the organic substance generation portion 3 even without providing a separate cooling device.

### <Post-stage treatment device>

Examples of the post-stage treatment device 13 that is disposed in the post-stage of the heat exchanger 20 in the thermally decomposed gas purification/cooling device 2 include a gas cooling tower, a filtration-type dust collector, a scrubber, an oil scrubber, a moisture separator including a gas chiller or the like, a low-temperature separation type (deep cooling type) separator, a fine particle separator composed of a variety of filters, a desulfurization device (sulfide separator), a film separation type separator, a deoxidation device, a pressure swing adsorption type separator (PSA), a temperature swing adsorption type separator (TSA), a pressure/temperature swing adsorption type separator (PTSA), a separator in which activated carbon is used, a separator in which a deoxidation catalyst, specifically, a copper catalyst or a palladium catalyst is used, a shift reactor and the like. One of these may be used singly or two or more may be jointly used.

Next, an example of the post-stage treatment device 13 will be described in more detail using Fig. 2. The post-stage treatment device 13 shown in Fig. 2 includes a gas cooling tower 21 that is disposed in the post-stage of the heat exchanger 20, a filtration-type dust collector 22 that is disposed in the post-stage of the gas cooling tower 21 and a scrubber 23 that is disposed in the post-stage of the filtration-type dust collector 22.

In addition, the post-stage treatment device 13 further includes other treatment devices (not shown) in the post-stage of the scrubber 23, and the synthesis gas G2 discharged from the scrubber 23 may be treated (purified, cooled or the like) as appropriate with the treatment devices in the post-stage.

In a case where the thermally decomposed gas purification/cooling device 2 includes the post-stage treatment device 13 shown in Fig. 2, the heat exchanger 20 cools the synthesis gas G2 to a temperature of, for example, 100°C or higher and 300°C or lower, preferably 120°C or higher and 240°C or lower and more preferably 140°C or higher and 200°C or lower. In the case of further including the post-stage treatment device 13 shown in Fig. 2, since the gas cooling tower 21 is disposed in the post-stage of the heat exchanger 20, when the temperature of the synthesis gas G2 that is cooled in the heat exchanger 20 is set within the above-described range, the synthesis gas G2 is supplied at a relatively low temperature to the gas cooling tower 21, which makes it unnecessary to excessively cool the synthesis gas G2 in the gas cooling tower 21. Therefore, it is possible to decrease the amount of water that is sprayed to the synthesis gas G2 in the gas cooling tower 21, and furthermore, it becomes unnecessary to supply the synthesis gas G2 having a high water content rate to the filtration-type dust collector 22 and the scrubber 23. Therefore, it is possible to suppress the amount of water transferred to the scrubber 23 from the gas cooling tower 21 and to prevent water from excessively agglomerating in the filtration-type dust collector 22.

### «Gas cooling tower»

The gas cooling tower 21 is a facility that cools a gas that passes through the inside of the gas cooling tower 21 (synthesis gas G2) by water spray. The gas cooling tower 21 includes one or more water spray openings 24 for spraying water to the synthesis gas G2 on the inner peripheral surface. Two or more water spray openings 24 are preferably provided, and the two or more water spray openings 24 are more preferably provided at different height positions in the cooling tower 21. When a plurality of the water spray openings 24 is provided and, furthermore, the height positions thereof are different, it is possible to more sufficiently and efficiently cool the synthesis gas G2 by water spray.

In the gas cooling tower 21, it is preferable that the synthesis gas G2 is introduced from the upper portion side, the synthesis gas G2 is passed through the inside of the gas cooling tower 21 so as to form a descending current, and the synthesis gas G2 is cooled by water sprayed from the water spray openings 24 while passing through the inside of the gas cooling tower 21. In this case, the synthesis gas G2 is preferably discharged from the lower portion side of the gas cooling tower 21.

The temperature of the synthesis gas G2 that is introduced into the gas cooling tower 21 is 100°C or higher, but the water that is sprayed from the water spray openings 24 is lower than 100°C. Therefore, the synthesis gas G2 is cooled due to the temperature difference and is also cooled by the vaporization heat generated when the water sprayed from the water spray openings 24 vaporizes. A part of the vaporized water is preferably mixed into the synthesis gas G2 as water vapor. A part or all of the water that is sprayed from the water spray openings 24 may be in a vaporized state when sprayed.

In the gas cooling tower 21, the synthesis gas G2 is preferably cooled to a temperature of 100°C or higher and 200°C or lower and preferably discharged to the outside of the gas cooling tower 21 within the above-described temperature range. When the synthesis gas G2 is cooled to 200°C or lower, it is possible to purify the synthesis gas G2 in the filtration-type dust collector 22 to be described below without damaging the filtration-type dust collector 22 or degrading the dust collection performance. In addition, when the synthesis gas G2 is cooled to 100°C or higher, the majority of the sprayed water is vaporized and mixed into the synthesis gas G2. Therefore, in the gas cooling tower 21, a large amount of the sprayed water is not discharged, which makes it unnecessary to introduce a large drain facility into the gas cooling tower 21.

Here, a part of the water sprayed in the gas cooling tower 21 may drop downward in the gas cooling tower 21 as a liquid and be recovered. In addition, the solid impurities, such as char and tar, remaining in the synthesis gas G2 may also collide with the sprayed water and thereby drop downward and be recovered.

The synthesis gas G2 is preferably cooled in the gas cooling tower 21 to a temperature of more preferably 120°C or higher and 180°C or lower and still more preferably 130°C or higher and 170°C or lower, cooled to these temperatures and discharged to the outside. When the synthesis gas G2 is cooled to 120°C or higher, it is possible to prevent the water mixed into the synthesis gas G2 from liquefying in a large quantity in the gas cooling tower 21 and, furthermore, the filtration-type dust collector 22 to be described below. In addition, when the synthesis gas G2 is cooled to 180°C or lower, it becomes easy to further avoid the damage or functional degradation of the filtration-type dust collector 22.

### «Filtration-type dust collector»

The synthesis gas G2 cooled in the gas cooling tower 21 passes through the filtration-type dust collector 22. As the filtration-type dust collector 22, a dust collector called a so-called bag filter can be used, and the bag filter includes a casing and a filter medium accommodated in the casing. The filter medium is not particularly limited, and, for example, woven fabric such as a glass fiber and a PTFE fiber, felt or the like is used.

There is a case where the solid impurities such as tar and char that are not completely removed in the cyclone 11 remain in the synthesis gas G2; however, when the synthesis gas G2 passes through the filtration-type dust collector 22, the solid impurities are removed. When the solid impurity is removed, it is possible to prevent the sticking of the solid impurity in each device in the post-stage of the filtration-type dust collector 22. For example, in the organic substance generation portion 3, it is ordinary that gas is blown into a reactor through a sparger, and the sticking of the solid impurity in the sparger can be prevented. Furthermore, when the solid impurity is removed, it is easy to enhance the activity of the microbial catalyst in the organic substance generation portion 3, and it is possible to prevent the death of the microbial catalyst due to the influence of the impurity and to synthesize an organic substance at a high conversion efficiency.

When the synthesis gas G2 is cooled in the gas cooling tower 21 as described above, the temperature of the synthesis gas G2 at the time of passing through the filtration-type dust collector 22 also becomes a temperature of preferably 100°C or higher and 200°C or lower, more preferably 120°C or higher and 180°C or lower and still more preferably 130°C or higher and 170°C or lower. Therefore, it is possible to prevent the high-temperature synthesis gas G2 from damaging the filtration-type dust collector 22 or degrading the filtration performance. In addition, it is also possible to prevent the synthesis gas G2 that is contained in the synthesis gas G2 from liquefying in a large quantity in the filtration-type dust collector 22.

### <<Scrubber>>

The synthesis gas G2 cooled in the gas cooling tower 21 passes through the scrubber 23. In the present embodiment, the synthesis gas G2 cooled in the gas cooling tower 21 and discharged from the filtration-type dust collector 22 passes through the scrubber 23 that is disposed in the post-stage of the filtration-type dust collector 22. The synthesis gas G2 contains a variety of impurities other than the above-described solid impurity, and, for example, a water-soluble impurity is contained. Examples of the water-soluble impurity include acidic gases such as hydrogen sulfide, hydrogen chloride and blue acid, basic gases such as ammonia and oxides such as NOx and SOx. These water-soluble impurities are removed when passing through the scrubber 23.

In addition, the synthesis gas G2 also contains oil-based impurities such as BTEX (benzene, toluene, ethylbenzene and xylene), naphthalene, 1-naphthol and 2-naphthol, but these may also be removed appropriately in the scrubber 23, and the solid impurity or the like that could not be recovered in the filtration-type dust collector 22 may also be appropriately removed.

The scrubber 23 is not particularly limited as long as the scrubber 23 is configured to bring the synthesis gas G2 and water into contact with each other and is, for example shown in Fig. 2, preferably configured to bring water sprayed from a nozzle 25 provided in the upper portion (for convenience, also referred to as "washing water") into contact with the synthesis gas G2. In this case, scrubber 23 is preferably provided with an introduction path 27, a supply path 28, a discharge path 29 and the like. In addition, a storage portion 26 that stores the washing water is provided in the lower portion of the scrubber 23. The washing water stored in the storage portion 26 may be appropriately stirred with a stirring device, not shown.

The introduction path 27 is a path for introducing the synthesis gas G2 into the scrubber 23, and an introduction opening 27A of the introduction path 27 is provided, for example, above the liquid surface of the washing water stored in the storage portion 26 in the scrubber 23.

The supply path 28 supplies the washing water such that the water is circulated in the scrubber 23 and brought into contact with the synthesis gas G2. Specifically, the supply path 28 sprays the washing water stored in the storage portion 26 downward in the scrubber 23 from the nozzle 25 to come into contact with the synthesis gas G2. Here, for example, a pump (not shown) is provided in the supply path 28, and the washing water is pneumatically sent to the nozzle 25 by the pump. In addition, the washing water is sprayed downward from the nozzle 25 in the scrubber 23. The discharge path 29 is provided in the upper portion of the scrubber 23 and discharges the synthesis gas G2 that has come into contact with the washing water sprayed from the nozzle 25 to the outside.

The washing water that is used in the scrubber 23 may be water alone or a chemical may be added thereto as appropriate.

Furthermore, a removal device 19 may be provided in the scrubber 23. The removal device 19 is a device for removing, for example, the impurities that are contained in the washing water (the oil-based impurities, the solid impurity, the water-soluble impurities and the like). The removal device 19 is preferably provided on a circulation path that circulates the water in the storage portion 26, for example. The removal device 19 preferably removes, for example, the oil-based impurities that are contained in the washing water, the solid impurity that does not dissolve in the washing water, the water-soluble impurities that dissolve in the washing water and the like. Therefore, the removal device 19 may be an oil-water separator or the like, may be a filter or the like that removes the solid impurity, may be a combination of two or more of these and may have any configuration as long as the impurities that are contained in the washing water can be removed. With providing the removal device 19, the scrubber 23 prevents the accumulation of the impurities in the washing water.

The synthesis gas G2 is preferably cooled by coming into contact with water in the scrubber 23. As described above, the synthesis gas G2 is cooled in the gas cooling tower 21 and introduced into the scrubber 23 in a state of being cooled to a predetermined temperature (a temperature of preferably 100°C or higher and 200°C or lower, more preferably 120°C or higher and 180°C or lower and still more preferably 130°C or higher to 170°C).

Incidentally, the temperature of the water that comes into contact with the synthesis gas G2 in the scrubber 23 is lower than 100°C, preferably 0°C or higher and 40°C or lower and more preferably 5°C or higher and 30°C or lower.

As "the temperature of the water that comes into contact with the synthesis gas G2" in the present specification, in a case where the washing water is circulated and brought into contact with the synthesis gas G2 as described above, the temperature of the water immediately before coming into contact with the synthesis gas G2, that is, the water (washing water) sprayed from the nozzle 25 may be measured. In addition, in a case where the synthesis gas G2 is introduced into the stored water (washing water) as described below, the temperature of the washing water stored in the storage portion 26 may be measured.

The synthesis gas G2 comes into contact with the water having the above-described temperature in the scrubber 23 and is thereby cooled to a temperature of, for example, lower than 100°C, preferably 40°C or lower and more preferably 38°C or lower. When the synthesis gas G2 is cooled to a predetermined temperature that is lower than the boiling point of water in the scrubber 23 as described above, at least a part of water mixed into the synthesis gas G2 in the gas cooling tower 22 (water vapor) is condensed and removed. Therefore, it becomes possible to appropriately remove water even without separately providing a large device for removing the water mixed in the gas cooling tower 22. In addition, the synthesis gas G2 is cooled to 40°C or lower, it is possible to supply the synthesis gas G2 having an appropriate temperature to the organic substance generation portion 3 even without separately providing a cooling device. In addition, even in a case where a cooling device is included in a treatment device that is provided in the post-stage of the scrubber 23, it is possible to reduce the load in the cooling device.

The synthesis gas G2 is preferably cooled to a temperature of, for example, 0°C or higher by coming into contact with water and is preferably cooled to a temperature of 5°C or higher.

It is preferable that the scrubber 23 is provided with a temperature controller, not shown, and the temperature of the washing water is controlled with the temperature controller. The temperature controller may be attached to, for example, the supply path 28 to adjust the temperature of the washing water that passes through the inside of the supply path 28 or may be provided on the outer periphery of the scrubber 23 to adjust the temperature of the washing water stored in the storage portion 26 in the scrubber 23. The temperature controller preferably puts the temperature of the washing water that passes through the supply path 28 or the washing water stored in the storage portion 26 into the above-described range by cooling or the like. In addition, the temperature of the water that is brought into contact with the synthesis gas G2 may be maintained within a certain temperature range by appropriately replacing the water that is stored in the storage portion 26.

In the above description, an aspect in which the synthesis gas G2 comes into contact with the washing water that is sprayed from the nozzle 25 in the scrubber 23 has been described, but the synthesis gas G2 may be introduced into the washing water that is stored in the storage portion 26.

In this case, the supply path 28 and the nozzle 25 are not provided, and the washing water is not sprayed from the nozzle. In addition, the introduction opening 27A of the introduction path 27 is disposed below the liquid surface of the washing water stored in the storage portion 26. The synthesis gas G2 comes into contact with the washing water stored in the storage portion 26, whereby the synthesis gas G2 is washed and preferably cooled.

Even in a case where the synthesis gas G2 is introduced into the washing water that is stored in the storage portion 26, the temperature of the water that comes into contact with the synthesis gas G2 or the temperature of the synthesis gas G2 (that is, the temperature of the synthesis gas G2 that is introduced into the scrubber 23 or the temperature of the cooled synthesis gas G2) is as described above.

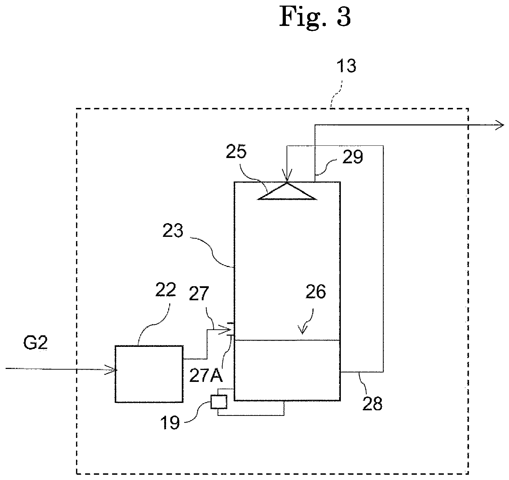

Next, a first modification example of the post-stage treatment device 13 will be described in more detail using Fig. 3. The post-stage treatment device 13 shown in Fig. 3 includes the filtration-type dust collector 22 that is disposed in the post-stage of the heat exchanger 20 and the scrubber 23 that is disposed in the post-stage of the filtration-type dust collector 22. That is, in the above-described embodiment, a configuration in which the gas cooling tower 21 is provided has been described, but the gas cooling tower 21 may not be provided. In a case where the gas cooling tower 21 is not provided, the synthesis gas G2 that has passed at least the filtration-type dust collector 22 and the scrubber 23 is brought into contact with the microbial catalyst and converted to an organic substance in the organic substance generation portion 3. The synthesis gas G2 to be supplied to the filtration-type dust collector 22 and the scrubber 23 has passed through the cyclone 11, whereby the dust component has been separated and removed in the cyclone 11, and thus the load of a purification treatment in each of the filtration-type dust collector 22 and the scrubber 23 is maintained at a low level.

In addition, the synthesis gas G2 discharged from the scrubber 23 may be further purified with a device other than the post-stage treatment device 13.

In a case where the thermally decomposed gas purification/cooling device 2 includes the post-stage treatment device 13 shown in Fig. 3, the cooling temperature in the heat exchanger 20 is, for example, 100°C or higher and 200°C or lower, preferably 120°C or higher and 180°C or lower and more preferably 130°C or higher and 170°C or lower. In the case of including the post-stage treatment device 13 shown in Fig. 3, since the filtration-type dust collector 22 is disposed in the post-stage of the heat exchanger 20, it is necessary to cool the synthesis gas G2 to the heatproof temperature of the filtration-type dust collector 22 or lower in the heat exchanger 20, and, when the temperature of the synthesis gas G2 that is cooled in the heat exchanger 20 is set within the above-described range, it is possible to prevent the high-temperature synthesis gas G2 from damaging the filtration-type dust collector 22 or degrading the filtration performance.

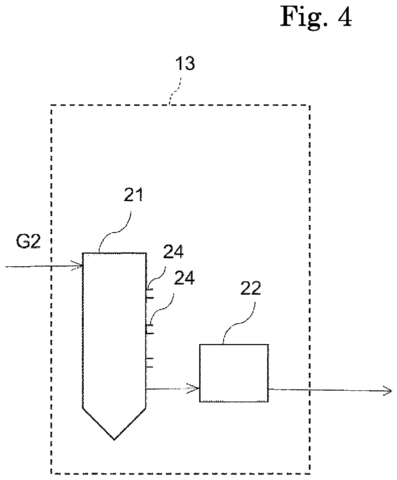

Next, a second modification example of the post-stage treatment device 13 will be described in more detail using Fig. 4. The post-stage treatment device 13 shown in Fig. 4 includes the gas cooling tower 21 that is disposed in the post-stage of the heat exchanger 20 and the filtration-type dust collector 22 that is disposed in the post-stage of the gas cooling tower 21. That is, in the above-described embodiment, a configuration in which the scrubber 23 is provided has been described, but the scrubber 23 may not be provided. In a case where the scrubber 23 is not provided, the synthesis gas G2 that has passed at least the gas cooling tower 21 and the filtration-type dust collector 22 is brought into contact with the microbial catalyst and converted to an organic substance in the organic substance generation portion 3. The synthesis gas G2 that is discharged from the filtration-type dust collector 22 in the present embodiment typically has a relatively high temperature (for example, 100°C or higher); however, in a case where the scrubber 23 is not provided, it is preferable that a cooling device other than the scrubber 23 is provided in the post-stage of the filtration-type dust collector 22 and the synthesis gas G2 discharged from the filtration-type dust collector 22 is cooled with the cooling device other than the scrubber 23.

In addition, in a case where the scrubber 23 is not provided, in addition to the cooling device, one or more treatment devices selected from the above-described post-stage treatment devices may be provided in the post-stage of the filtration-type dust collector 22 and the synthesis gas G2 discharged from the filtration-type dust collector 22 may be treated as appropriate with the post-stage treatment device.

In addition, in a case where there is no need to purify the organic substance produced in the organic substance generation portion 3, in a case where there is no need to separate water from an organic substance-containing liquid or the like, the separator 31 may not be provided.

In addition, the synthesis gas G2 discharged from the filtration-type dust collector 22 may be further purified with the treatment device disposed in the post-stage of the filtration-type dust collector 22.

In a case where the thermally decomposed gas purification/cooling device 2 includes the post-stage treatment device 13 shown in Fig. 4, the cooling temperature in the heat exchanger 20 is, for example, 150°C or higher and 300°C or lower, preferably 170°C or higher and 280°C or lower and more preferably 190°C or higher and 260°C or lower. In the case of including the post-stage treatment device 13 shown in Fig. 4, since the gas cooling tower 21 is disposed in the post-stage of the heat exchanger 20, when the cooling temperature is within the above-described range, the synthesis gas G2 is supplied at a relatively low temperature to the gas cooling tower 21, and it is unnecessary to excessively cool the synthesis gas G2 in the gas cooling tower 21. Therefore, it is possible to decrease the amount of water that is sprayed to the synthesis gas G2 in the gas cooling tower 21, and furthermore, it becomes unnecessary to supply the synthesis gas G2 having a high water content rate to the filtration-type dust collector 22. Therefore, it is possible to prevent water from excessively agglomerating in the filtration-type dust collector 22.

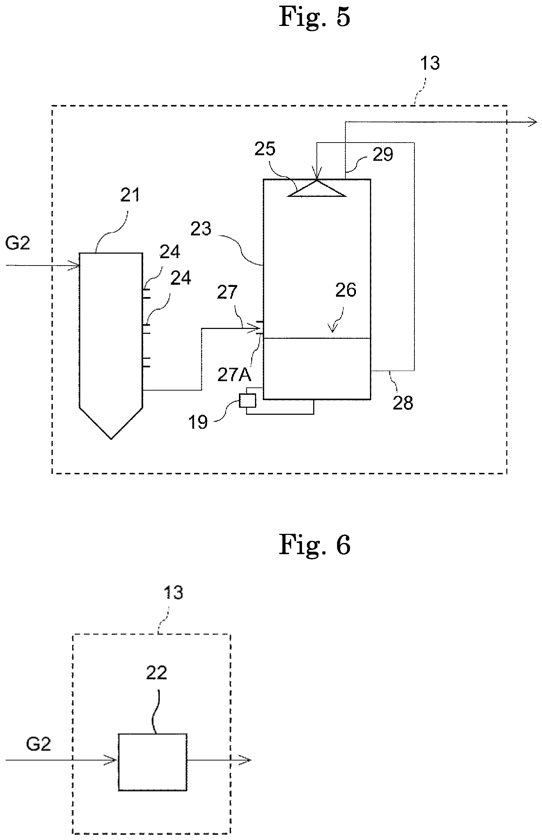

Next, a third modification example of the post-stage treatment device 13 will be described in more detail using Fig. 5. The post-stage treatment device 13 shown in Fig. 5 includes the gas cooling tower 21 that is disposed in the post-stage of the heat exchanger 20 and the scrubber 23 that is disposed in the post-stage of the gas cooling tower 21. That is, in the above-described embodiment, a configuration in which the filtration-type dust collector 22 is provided has been described, but the filtration-type dust collector 22 may not be provided. When the filtration-type dust collector 22 is not provided, the synthesis gas G2 cooled in the gas cooling tower 21 is supplied to the scrubber 23 without passing through the filtration-type dust collector 22; however, since the synthesis gas G2 has passed through the cyclone 11, the dust component has been separated and removed in the cyclone 11, and thus there is no problem.

The synthesis gas G2 discharged from the scrubber 23 may be further purified with a device other than the post-stage treatment device 13.

In a case where the thermally decomposed gas purification/cooling device 2 includes the post-stage treatment device 13 shown in Fig. 5, the cooling temperature in the heat exchanger 20 is, for example, 200°C or higher and 300°C or lower, preferably 210°C or higher and 290°C or lower and more preferably 220°C or higher and 280°C or lower. In the case of including the post-stage treatment device 13 shown in Fig. 5, since the gas cooling tower 21 is disposed in the post-stage of the heat exchanger 20, when the cooling temperature is within the above-described range, the synthesis gas G2 is supplied at a relatively low temperature to the gas cooling tower 21, and it is unnecessary to excessively cool the synthesis gas G2 in the gas cooling tower 21. Therefore, it is possible to decrease the amount of water that is sprayed to the synthesis gas G2 in the gas cooling tower 21, and furthermore, it becomes unnecessary to supply the synthesis gas G2 having a high water content rate to the scrubber 23. Therefore, it is possible to suppress the amount of water transferred to the scrubber 23 from the gas cooling tower 21.

Next, a fourth modification example of the post-stage treatment device 13 will be described in more detail using Fig. 6. The post-stage treatment device 13 shown in Fig. 6 includes the filtration-type dust collector 22 that is disposed in the post-stage of the heat exchanger 20. That is, in the above-described embodiment, a configuration in which the gas cooling tower 21 and the scrubber 23 are provided has been described, but the gas cooling tower 21 and the scrubber 23 may not be provided.

The synthesis gas G2 discharged from the filtration-type dust collector 22 may be further purified with a device other than the post-stage treatment device 13.

In a case where the thermally decomposed gas purification/cooling device 2 includes the post-stage treatment device 13 shown in Fig. 6, the heat exchanger 20 cools the synthesis gas G2 to a temperature of, for example, 30°C or higher and 60°C or lower, preferably 35°C or higher and 55°C or lower and more preferably 40°C or higher and 50°C or lower. In the case of including the post-stage treatment device 13 shown in Fig. 6, since the filtration-type dust collector 22 is disposed in the post-stage of the heat exchanger 20, and other cooling devices are not provided, when the temperature of the synthesis gas G2 that is cooled in the heat exchanger 20 is set within the above-described range, it is possible to prevent the high-temperature synthesis gas G2 from damaging the filtration-type dust collector 22 or degrading the filtration performance. In addition, it is easy to enhance the activity of the microbial catalyst in the organic substance generation portion 3 in the post-stage of the post-stage treatment device 13, and it is also possible to prevent the death of the microbial catalyst due to the influence of the impurities.

In the above description, several modification examples of the configuration of the post-stage treatment device 13 have been described, but the configuration is not limited thereto, and the configuration simply needs to be capable of supplying the synthesis gas G2 suitable for the organic substance generation portion 3 that is disposed in the post-stage. For example, in a case where the purification treatment of the synthesis gas G2 with the cyclone 11 is sufficient, among the gas cooling tower 21, the filtration-type dust collector 22 and the scrubber 23, only the gas cooling tower 21 may be provided in the configuration. In addition, among the gas cooling tower 21, the filtration-type dust collector 22 and the scrubber 23, only the scrubber 23 may be provided in the configuration. Furthermore, in a case where the purification treatment and the cooling treatment of the synthesis gas G2 with the cyclone 11 and the heat exchanger 20 are sufficient, all of the gas cooling tower 21, the filtration-type dust collector 22 and the scrubber 23 may not be provided in the configuration as the post-stage treatment device 13.

### <Organic substance generation portion>

As described above, the synthesis gas G2 that has passed through at least the cyclone 11 and the heat exchanger 20 in the thermally decomposed gas purification/cooling device 2 is supplied to the organic substance generation portion 3 as shown in Fig. 1. The synthesis gas G2 that is supplied to the organic substance generation portion 3 is preferably the synthesis gas G2 that has passed through the heat exchanger 20, the gas cooling tower 21, the filtration-type dust collector 22 and the scrubber 23 in this order. In the organic substance generation portion 3, the synthesis gas G2 is brought into contact with the microbial catalyst to generate an organic substance. As the microbial catalyst, a gas-assimilating microbial is preferably used.

The organic substance generation portion 3 includes a fermenter (reactor) filled with a culture containing water and the microbial catalyst. The synthesis gas G2 is supplied to the inside of the fermenter, and the synthesis gas G2 is converted to an organic substance in the fermenter. The organic substance preferably contains any of ethanol and isopropanol and more preferably contains ethanol.

As the fermenter, a continuous fermenting device is preferably used, and any of a stirring type, an air lift type, a cell tower type, a loop type, an open bond type and a photobio type may be used.

The synthesis gas G2 and the culture may be continuously supplied to the fermenter, but there is no need to supply the synthesis gas G2 and the culture at the same time, and the synthesis gas G2 may be supplied to the fermenter to which the culture has been supplied in advance. Ordinarily, the synthesis gas G2 is blown into the fermenter through a sparger or the like.

A culture medium that is used when the microbial catalyst is cultured is not particularly limited as long as the composition is appropriate depending on germs and is a liquid containing water, which is a main component, and nutrients (for example, vitamins, phosphoric acid and the like) dissolved or distributed in this water.

In the organic substance generation portion 3, an organic substance is generated due to the microbial fermentation of the microbial catalyst and an organic substance-containing liquid is obtained.

The temperature of the fermenter is preferably controlled to 40°C or lower. When the temperature of the fermenter is controlled to 40°C or lower, the microbial catalyst in the fermenter does not die, and the synthesis gas G2 comes into contact with the microbial catalyst, whereby an organic substance such as ethanol is efficiently generated.

The temperature of the fermenter is more preferably 38°C or lower. In addition, in order to enhance the catalytic activity, the temperature is preferably 10°C or higher, more preferably 20°C or higher and still more preferably 30°C or higher.

### <Separator>

The device for producing an organic substance 1 includes a separator 31 that separates at least water from the organic substance-containing liquid.

As the separator 31, a distillation device 33 is preferably included, and a solid-liquid separator 32 is more preferably included in the pre-stage of the distillation device 33. As the separator 31, the solid-liquid separator 32 and the distillation device 33 are still more preferably used in combination. Hereinafter, a separation step that is carried out by combining the solid-liquid separator 32 and the distillation device 33 will be specifically described.

### «solid-liquid separator»

The organic substance-containing liquid obtained in the organic substance generation portion 3 is preferably separated into a solid component mainly containing a microbial and a liquid component containing the organic substance in the solid-liquid separator 32. The organic substance-containing liquid obtained in the organic substance generation portion 3 contains, in addition to the organic substance, which is a target substance, the microbial that was contained in the fermenter, the carcass thereof or the like and is thus separated into solid and liquid to remove these. As the solid-liquid separator 32, there are a filter, a centrifuge, devices in which a solution precipitation method is used and the like. In addition, the solid-liquid separator 32 may be a device that separates the liquid component containing the organic substance from the solid component by evaporating the liquid component from the organic substance-containing liquid (for example, a heated-air dryer). At this time, the liquid component containing the organic substance, which is the target substance, may be fully evaporated or the liquid component may be partially evaporated such that the organic substance, which is the target, is preferentially evaporated.

### <<Distillation device>>

The distillation device 33 carries out distillation for separating the organic substance, which is the target substance. The distillation device 33 is capable of purifying a large amount of the organic substance to a high purity by simple operation by means of separation by distillation. In the separation step that is carried out with the distillation device 33 in combination with the solid-liquid separator 32, distillation for further separating the organic substance, which is the target substance, from a liquid component separated with the solid-liquid separator 32 is carried out in the distillation device 33, whereby a large amount of the organic substance can be purified to a higher purity.

As the distillation device 33, a well-known distillation tower can be used. In addition, the distillation needs to be operated such that, for example, the organic substance, which is the target substance, (for example, ethanol) is contained in the distillate at a high purity and water is contained in the bottom product (that is, the distillation residue) as a main component (for example, 70 mass% or more and preferably 90 mass% or more). Such operation makes it possible to generally separate the organic substance, which is the target substance, and water.

The temperature in the distillation device 33 at the time of the distillation of the organic substance (for example, ethanol and isopropanol) is not particularly limited, but is preferably 100°C or lower and more preferably approximately 70°C to 95°C. When the temperature in the distillation device 33 is set within the above-described range, it is possible to reliably separate the required organic substance and the other components such as water.

The pressure in the distillation device 33 at the time of the distillation of the organic substance may be a normal pressure, but is preferably lower than the atmospheric pressure and more preferably approximately 60 to 150 kPa (gauge pressure). When the pressure in the distillation device 33 is set within the above-described range, it is possible to improve the separation efficiency of the organic substance and to improve the yield of the organic substance.

In the distillation device 33, the heat energy obtained from the synthesis gas G2 with the above-described heat exchanger 20 is preferably used for distillation. When the heat energy obtained from the synthesis gas G2 in the heat exchanger 20 is reused in the distillation device 33, it is possible to increase the temperature in the distillation device 33 at the time of the distillation of the organic substance. When the heat energy obtained from the synthesis gas G2 in the heat exchanger 20 is reused in the distillation device 33, it is possible to reduce the amount of energy used in the entire production process of the organic substance. The heat energy obtained from the synthesis gas G2 in the heat exchanger 20 can be transmitted through a heat energy path 33a connected to the heat exchanger 20 and the distillation device 33. The heat energy path 33a is not particularly limited and may have any configuration by which the heat energy of the synthesis gas G2 is transferred from the heat exchanger 20 to the distillation device 33 with the heat medium. The heat medium may be any of gas or liquid and may be a heat medium accompanying a phase change between gas and liquid. In addition, as described above, the heat exchanger 20 is preferably a boiler, and thus the heat medium is preferably water vapor. The use of water vapor as the heat medium makes it easy to reuse the heat energy of the synthesis gas G2. In a case where water vapor is used as the heat medium, some of the water vapor may be liquefied.

The water separated in the separator 31 is preferably reused and more preferably supplied to the gas cooling tower 21 and used for water spray in the gas cooling tower 21. When the water is reused as described above, the water that became unnecessary in the organic substance generation portion 3 does not become drainage water, which is preferable from the viewpoint of the environmental protection and the viewpoint of the economic efficiency. In addition, in the device for producing an organic substance 1, the separator 31 and the gas cooling tower 21 may be connected to each other and a water supply path 31a that supplies the water obtained in the separator 31 to the gas cooling tower 21 may be provided. The water supply path 31a is not particularly limited, but is preferably made of a pipe or the like. In addition, the water separated in the separator 31 may be supplied to the gas cooling tower 21 after being further purified to have a higher purity.

As described above, according to the present embodiment, the dust component is separated and removed from the thermally decomposed gas G1 with the cyclone 11, whereby the content of the dust component in the thermally decomposed gas G1 can be reduced, and it is possible to prevent a decrease in cooling efficiency or blockage of a gas flow path in the post-stage, which arises from the dust component.

In addition, according to the present embodiment, it is possible to use the heat energy obtained from the synthesis gas G2 with the heat exchanger 20 to increase the temperature in the distillation device 33 at the time of the distillation of the organic substance. Therefore, it is possible to reduce the amount of energy that is procured from outside during the distillation with the distillation device 33 and to reduce the amount of energy used in the entire production process of the organic substance.

According to the present embodiment, the cyclone 11 is provided in the post-stage of the gasification furnace 10, and thus the synthesis gas G2 to be supplied to the heat exchanger 20 has passed through the cyclone 11. Therefore, the dust component has been separated and removed in the cyclone 11, and enlargement by the dust component can be suppressed even when the synthesis gas G2 is cooled to 240°C or lower in the heat exchanger 20. In addition, the synthesis gas G2 is cooled in the heat exchanger 20 to 100°C or lower, which makes it possible not to provide some of the cooling devices in the post-stage. In addition, the synthesis gas G2 is cooled to approximately 40°C in the heat exchanger 20, whereby it is possible to supply the synthesis gas G2 at an appropriate temperature for the microbial catalyst to the organic substance generation portion 3 even without providing a separate cooling device.

In the above-described embodiment, an aspect in which the thermally decomposed gas G1 is obtained from waste in the gasification furnace 10 has been described, but the thermally decomposed gas G1 may be generated from a substance other than waste in the gasification furnace 10. For example, the thermally decomposed gas G1 may be generated from fossil resources such as natural gas, coal, heavy oil, petroleum discharged gas and oil shale, biomass other than waste or the like. In addition, the thermally decomposed gas G1 may be a gaseous by-product in a variety of production processes such as a steel production process, and, for example, the gasification furnace 10 may configure a steel production facility or the like.

### (Second embodiment)

Next, a second embodiment of the present invention will be described. Hereinafter, regarding the second embodiment, the same portions as in the first embodiment will not be described, and only differences from the first embodiment will be described.

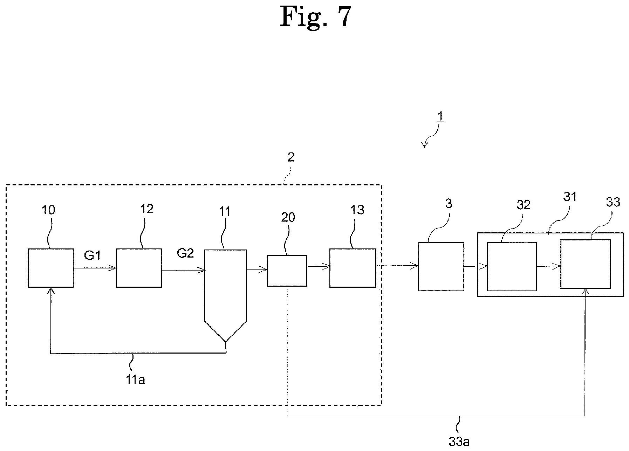

A difference of a device for producing an organic substance 1 in the second embodiment is that the reforming furnace 12 is disposed in the pre-stage of the cyclone 11 as shown in Fig. 7.

The cyclone 11 in the second embodiment is a device in which the synthesis gas G2 obtained in the reforming furnace 12 is introduced, the synthesis gas G2 in which solid and liquid are present in a mixed manner is swirled to generate a centrifugal force, and dust components such as tar and char, which are solid, are separated and removed by the centrifugal force. The dust component is separated and removed from the synthesis gas G2 with the cyclone 11, whereby the content of the dust component in the synthesis gas G2 can be reduced, and it is possible to prevent a decrease in cooling efficiency or blockage of a gas flow path in the post-stage, which arises from the dust component. The synthesis gas G2 from which the dust component has been separated and removed with the cyclone 11 is supplied to the heat exchanger 20.

In the above description, the gasification furnace 10 and the reforming furnace 12 have been described as mutually different members, but the gasification furnace 10 and the reforming furnace 12 may be integrated into a device, and the type of a gasifier is not limited as long as the synthesis gas G2 can be generated.

According to the device for producing an organic substance and a method for producing an organic substance according to the second embodiment of the present invention, equivalent effects described in the device for producing an organic substance and the method for producing an organic substance according to the first embodiment can be exhibited.

### (Third embodiment)

Next, a third embodiment of the present invention will be described. Hereinafter, regarding the third embodiment, the same portions as in the first embodiment will not be described, and only differences from the first embodiment will be described.

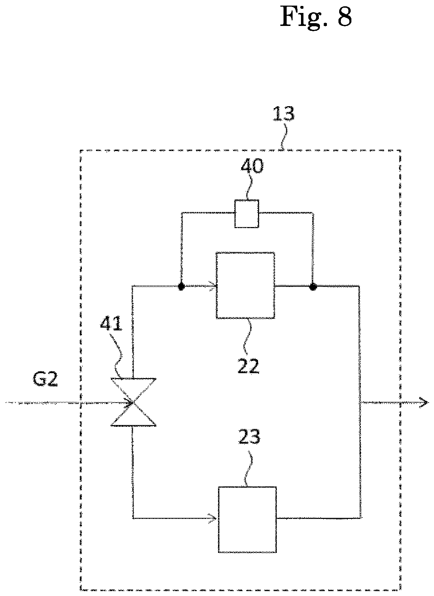

A difference of a device for producing an organic substance 1 in the third embodiment is that, as shown in Fig. 8, the filtration-type dust collector 22 and the scrubber 23 are provided as the post-stage treatment device 13 and the filtration-type dust collector 22 and the scrubber 23 are disposed in parallel in the post-stage of the heat exchanger 20. That is, in the device for producing an organic substance 1 in the third embodiment, the synthesis gas G2 discharged from the heat exchanger 20 is supplied to any of the filtration-type dust collector 22 and the scrubber 23 and purified.

In the case of including the post-stage treatment device 13 shown in Fig. 8, the cooling temperature in the heat exchanger 20 is, for example, 100°C or higher and 200°C or lower, preferably 120°C or higher and 180°C or lower and more preferably 130°C or higher and 170°C or lower. In the case of including the post-stage treatment device 13 shown in Fig. 8, since the filtration-type dust collector 22 is disposed in the post-stage of the heat exchanger 20, it is necessary to cool the synthesis gas G2 to the heatproof temperature of the filtration-type dust collector 22 or lower in the heat exchanger 20, and, when the temperature of the synthesis gas G2 that is cooled in the heat exchanger 20 is set within the above-described range, it is possible to prevent the high-temperature synthesis gas G2 from damaging the filtration-type dust collector 22 or degrading the filtration performance.

The device for producing an organic substance 1 in the third embodiment further includes a differential pressure-measuring device 40 and a flow path-switching portion 41.

,

### <Differential pressure-measuring device>

The differential pressure-measuring device 40 measures the differential pressure between the pre-stage and the post-stage of the filtration-type dust collector 22. The differential pressure between the synthesis gas G2 that is supplied to the filtration-type dust collector 22 and the synthesis gas G2 that is discharged from the filtration-type dust collector 22 is measured with the differential pressure-measuring device 40, and, in a case where the differential pressure exceeds a predetermined pressure (standard value), it is determined that blockage occurs in the filtration-type dust collector 22. In a case where the differential pressure does not exceed the standard value based on the measurement result of the differential pressure-measuring device 40, as the normal operation of the device for producing an organic substance 1, the synthesis gas G2 is supplied to the filtration-type dust collector 22, and the synthesis gas G2 is purified with the filtration-type dust collector 22. On the other hand, in a case where the differential pressure exceeds the standard value based on the measurement result of the differential pressure-measuring device 40, in order to carry out maintenance for eliminating blockage of the filtration-type dust collector 22, the supply of the synthesis gas G2 to the filtration-type dust collector 22 is stopped, and the synthesis gas G2 is supplied to the scrubber 23.

The standard value in the differential pressure-measuring device 40 refers to a value that is displayed by the differential pressure-measuring device 40 in a case where the synthesis gas G2 is being purified as appropriate with the filtration-type dust collector 22.

As the differential pressure-measuring device 40, it is possible to use a well-known measuring device capable of measuring the differential pressure between the pre-stage and the post-stage of the filtration-type dust collector 22.

### <Flow path-switching portion>

The device for producing an organic substance 1 includes the flow path-switching portion 41 that selectively switches the supply destination to the filtration-type dust collector 22 or the scrubber 23 through which the synthesis gas G2 is to be passed. The flow path-switching portion 41 can be configured using, for example, a two-way switching valve, a three-way switching valve or the like.

The flow path-switching portion 41 selectively switches the supply destination of the synthesis gas G2 discharged from the heat exchanger 20 to any of the filtration-type dust collector 22 or the scrubber 23. For example, when the device for producing an organic substance 1 is in normal operation, the flow path-switching portion 41 selects the filtration-type dust collector 22 as the supply destination of the synthesis gas G2. When the device for producing an organic substance 1 is in normal operation, as described above, the filtration-type dust collector 22 is selected as the supply destination of the synthesis gas G2, whereby it is possible to stop the operation of the scrubber 23, drainage from the scrubber 23 does not occur, and a problem of the drainage treatment cost or the environmental load can be eliminated.

In addition, as in a case where the device for producing an organic substance 1 is not in normal operation, for example, when blockage occurs in the filtration-type dust collector 22, the flow path-switching portion 41 selects the scrubber 23 as the supply destination of the synthesis gas G2. When the device for producing an organic substance 1 is not in normal operation, the use of the scrubber 23 makes it possible to continuously operate the device for producing an organic substance 1 without stopping the generation of the synthesis gas G2, and thus it is possible to prevent a decrease in production efficiency. In addition, in a case where the microbial catalyst is used in the organic substance generation portion 3, the device for producing an organic substance 1 can be continuously operated, whereby it is possible to continuously supply the purified synthesis gas G2 to the organic substance generation portion 3 and to prevent the death of the microbial catalyst.

The flow path-switching portion 41 may select the supply destination of the synthesis gas G2 discharged from a thermally decomposed gas purification/cooling device 2 depending on the measurement result by the differential pressure-measuring device 40. Specifically, in a case where the differential pressure between the pre-stage and the post-stage of the filtration-type dust collector 22 is measured with the differential pressure-measuring device 40 and the differential pressure does not exceed the standard value, the flow path-switching portion 41 supplies the synthesis gas G2 to the filtration-type dust collector 22. On the other hand, in a case where the differential pressure between the pre-stage and the post-stage of the filtration-type dust collector 22 is measured with the differential pressure-measuring device 40 and the differential pressure exceeds the standard value, it is determined that blockage occurs in the filtration-type dust collector 22, and the flow path-switching portion 41 supplies the synthesis gas G2 to the scrubber 23.

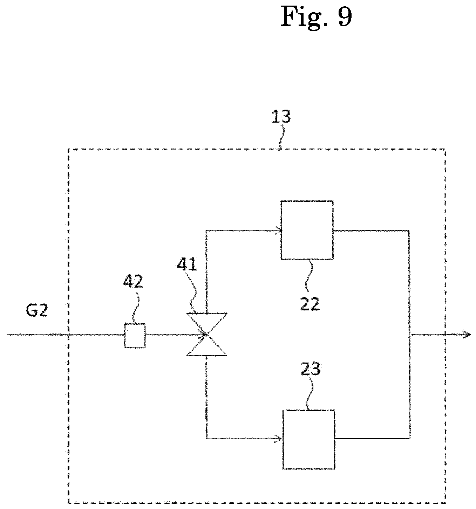

Next, a first modification example of the post-stage treatment device 13 provided in the device for producing an organic substance 1 in the third embodiment will be described in more detail using Fig. 9. The post-stage treatment device 13 shown in Fig. 9 includes a concentration-measuring device 42.

### <Concentration-measuring device>

The concentration-measuring device 42 is a device that measures the concentration of an impurity in the synthesis gas G2 discharged from the heat exchanger 20 and measures, for example, the concentration of at least any selected from a phase-transitable impurity and a solid impurity in the synthesis gas G2.

In a case where the concentration of a specific impurity (for example, at least any selected from the phase-transitable impurity and the solid impurity) is measured with the concentration-measuring device 42 and the concentration of the impurity exceeds a predetermined value (standard value), it is determined that the concentration of the impurity exceeds the purification treatment capability in the filtration-type dust collector 22, and the synthesis gas G2 is supplied to the scrubber 23 in order to avoid blockage of the filtration-type dust collector 22.

Examples of the concentration of the specific impurity include the concentration of the phase-transitable impurity. The phase-transitable impurity refers to an impurity capable of phase transition between gaseous and solid phases, including a sublimable substance such as naphthalene, 1-naphthol and 2-naphthol. The concentration of the phase-transitable impurity may be the concentration of a specific component in the phase-transitable impurities or may be the concentration of the total amount of the phase-transitable impurities.

Examples of the concentration of the specific component in the phase-transitable impurity include the concentration of naphthalene. A synthesis gas derived from waste contains a large amount of naphthalene, and, when the concentration of naphthalene is representatively measured, it is possible to roughly grasp the concentration of the phase-transitable impurity that is contained in the entire synthesis gas.

As the standard value of the concentration of the phase-transitable impurity, the concentration of naphthalene is, for example, 500 ppm or more.

In addition, the concentration of the specific component may be the concentration of the total amount of two or more components such as naphthalene, 1-naphthol and 2-naphthol or the like.

Regarding the solid impurity as well, the concentration of the entire solid impurity may be measured or the concentration of a specific component in the solid impurities may be measured. As the concentration of the entire solid impurity, for example, the concentration of the entire solid impurity that is contained in the synthesis gas G2 adjusted to a predetermined temperature may be measured after the synthesis gas G2 is adjusted to the certain temperature. In addition, as the concentration of the specific component in the solid impurities, the concentration of pure tar, pure char, the total amount of tar and char or the like may be measured.

As the standard value of the concentration of the solid impurity, for example, the concentration of tar is 5 g/Nm³ or more, and the concentration of dust is 50 g/Nm³ or more. Here, these solid impurities and concentration ranges are simply examples, and other solid impurities and concentration ranges are also appliable.

Furthermore, the concentration of both the phase-transitable impurity and the solid impurity may be measured. Specifically, the concentration of both the phase-transitable impurity and the solid impurity may be measured or the concentration of the total amount may be measured. In addition, the concentration of both a specific component in the phase-transitable impurities and a specific component in the solid impurities may be measured. In the case of measuring the concentration of two or more components, while there are no particular limitations, for example, in a case where the concentration of one component among measured components exceeds the standard value, the synthesis gas G2 needs to be supplied to the scrubber 23.

Examples of the concentration-measuring device 42 include a variety of measuring devices such as a gas chromatography device, a mass spectrometer, a gas chromatography-mass spectrometer, a secondary ion mass spectrometer, an atomic absorption spectrometer, a Raman spectrophotometer and a Fouriertransform infrared spectrometer and the like.

In addition, as the concentration of the solid impurity, the solid impurity may be trapped with an adsorbent, a filter or the like maintained at a specific temperature and the trapped component may be measured with the above-described measuring device. Regarding the phase-transitable impurity as well, the phase-transitable impurity may be trapped with an adsorbent or the like and the trapped component may be measured with the above-described measuring device.

The flow path-switching portion 41 may select the supply destination of the synthesis gas G2 discharged from the heat exchanger 20 depending on the measurement result by the above-described concentration-measuring device 42. Specifically, in a case where the concentration of a specific impurity (for example, at least any selected from the phase-transitable impurity and the solid impurity) is measured with the concentration-measuring device 42 and the concentration does not exceed the standard value, it is determined that the concentration of the impurity is within the purification treatment capability in the filtration-type dust collector 22, and the flow path-switching portion 41 supplies the synthesis gas G2 to the filtration-type dust collector 22 as normal operation. On the other hand, in a case where the concentration of at least any of the phase-transitable impurity and the solid impurity is measured with the concentration-measuring device 42 and the concentration of at least any of the phase-transitable impurity and the solid impurity exceeds the standard value, it is determined that the concentration of the impurities exceeds the purification treatment capability in the filtration-type dust collector 22, and the flow path-switching portion 41 supplies the synthesis gas G2 to the scrubber 23 as abnormal operation in order to avoid blockage of the filtration-type dust collector 22.

As described above, in the present embodiment, the use of the measurement result of the concentration-measuring device 42 makes it possible to prevent blockage of the filtration-type dust collector 22 and to decrease drainage from the scrubber 23.

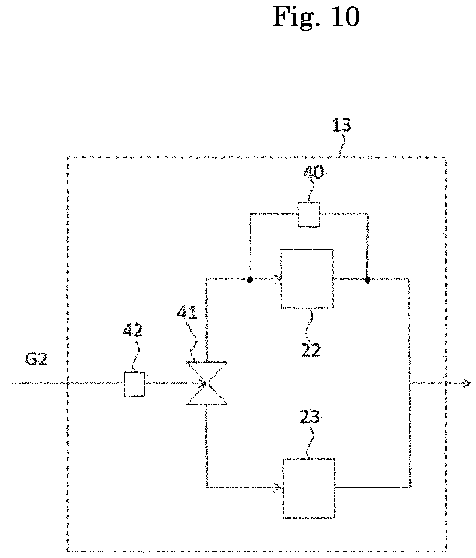

Next, a second modification example of the post-stage treatment device 13 provided in the device for producing an organic substance 1 in the third embodiment will be described in more detail using Fig. 10. In the post-stage treatment device 13 shown in Fig. 10, the concentration-measuring device 42 and the differential pressure-measuring device 40 are jointly provided.

In the post-stage treatment device 13 shown in Fig. 10, the concentration-measuring device 42 and the differential pressure-measuring device 40 are jointly provided, and it is possible to selectively switch the supply destination to the filtration-type dust collector 22 or the scrubber 23 through which the synthesis gas G2 is passed depending on the measurement results of the concentration-measuring device 42 and the differential pressure-measuring device 40.

In the present embodiment, first, the concentration of the impurity in the synthesis gas G2 discharged from the thermally decomposed gas purification/cooling device 2 is measured with the concentration-measuring device 42, in a case where the concentration of the impurity does not exceed the standard value, the synthesis gas G2 is supplied to the filtration-type dust collector 22, and, in a case where the concentration of the impurity exceeds the standard value, the synthesis gas G2 is supplied to the scrubber 23. That is, the use of the measurement result of the concentration-measuring device 42 makes it possible to prevent blockage of the filtration-type dust collector 22 and to decrease drainage from the scrubber 23.

In addition, in the present embodiment, in association with the measurement of the concentration with the concentration-measuring device 42, the differential pressure between the pre-stage and the post-stage of the filtration-type dust collector 22 is measured with the differential pressure-measuring device 40, whereby, even in a case where blockage of the filtration-type dust collector 22, which arises from a cause other than the concentration of the impurity in the synthesis gas G2 such as deterioration over time, occurs, it becomes possible to cope with the blockage by supplying the synthesis gas G2 to the scrubber 23, and the continuous operation of the device for producing an organic substance 1 becomes possible.

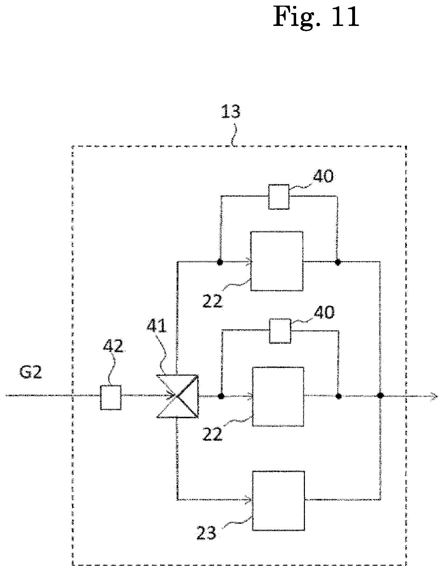

Next, a third modification example of the post-stage treatment device 13 provided in the device for producing an organic substance 1 in the third embodiment will be described in more detail using Fig. 11. In the post-stage treatment device 13 shown in Fig. 11, a plurality of the filtration-type dust collectors 22 is provided, and the plurality of filtration-type dust collectors 22 and the scrubber 23 are disposed in parallel in the post-stage of the thermally decomposed gas purification/cooling device 2.

In the post-stage treatment device 13 shown in Fig. 11, two filtration-type dust collectors 22 are disposed in parallel in the post-stage of the thermally decomposed gas purification/cooling device 2, and, when the device for producing an organic substance 1 is in normal operation, the synthesis gas G2 is supplied to at least any of the filtration-type dust collectors 22, and the synthesis gas G2 is purified.

In each of the filtration-type dust collectors 22, the differential pressure-measuring device 40 may be provided, and the differential pressure between the pre-stage and the post-stage of the filtration-type dust collector 22 may be measured. In a case where the differential pressure exceeds the standard value based on the measurement result of the differential pressure-measuring device 40, in order to carry out maintenance for eliminating blockage of one filtration-type dust collector 22, which has been the measuring object of the differential pressure, the supply of the synthesis gas G2 is stopped, and the synthesis gas G2 is supplied to the other filtration-type dust collector 22 or the synthesis gas G2 is supplied to the scrubber 23. At this time, the supply destination of the synthesis gas G2 is preferentially the other filtration-type dust collector 22, and the operation of the scrubber 23 is extremely decreased, whereby it is possible to eliminate a problem of the treatment cost for drainage from the scrubber 23 or the environmental load.

As the post-stage treatment device 13 shown in Fig. 11, a configuration in which the differential pressure-measuring device 40 and the concentration-measuring device 42 are jointly provided has been described, but the concentration-measuring device 42 may not be provided or the differential pressure-measuring device 40 may not be provided.

In the above-described embodiment, a form in which one or two filtration-type dust collectors 22 are provided has been described, but the form is not limited thereto, and three or more filtration-type dust collectors 22 may be provided and three or more filtration-type dust collectors 22 and scrubbers 23 may be disposed in parallel in the post-stage of the thermally decomposed gas purification/cooling device 2.

In addition, in the above-described embodiment, a configuration in which the differential pressure-measuring device 40 is provided as a single body, a configuration in which the concentration-measuring device 42 is provided as a single body and a configuration in which the differential pressure-measuring device 40 and the concentration-measuring device 42 are jointly provided have been described, but both the differential pressure-measuring device 40 and the concentration-measuring device 42 may not be provided. In a case where both the differential pressure-measuring device 40 and the concentration-measuring device 42 are not provided, the supply destination of the synthesis gas G2 is changed to the differential pressure-measuring device 20 or the concentration-measuring device 21 at certain intervals, whereby it is possible to reduce drainage from the scrubber 23 while suppressing blockage of the differential pressure-measuring device 20 and to efficiently remove the impurity from the synthesis gas G2 derived from waste.

In the above-described embodiment, the post-stage treatment device 13 is not limited to the above-described configurations, and each of the above-described devices listed as the post-stage treatment device 13 in the first embodiment may be provided as appropriate in the pre-stage of the flow path-switching portion 41 or in the post-stage of the filtration-type dust collector 22 and the scrubber 23.

In addition, in the present embodiment, the gas cooling tower 21 may be provided in the pre-stage of the flow path-switching portion 41 and in the post-stage of the heat exchanger 20. The behaviors of the heat exchanger 20 and the gas cooling tower 21 at this time are as described in the first embodiment.

According to the device for producing an organic substance and the method for producing an organic substance according to the third embodiment of the present invention, equivalent effects described in the device for producing an organic substance and the method for producing an organic substance according to the first embodiment can be exhibited.

In the above description of the third embodiment, an aspect in which the cyclone 11 is disposed in the pre-stage of the reforming furnace 12 has been described; however, in the third embodiment, the reforming furnace 12 may be disposed in the pre-stage of the cyclone 11 as described in the second embodiment.

### (Fourth embodiment)

Next, a fourth embodiment of the present invention will be described. Hereinafter, regarding the fourth embodiment, the same portions as in the first embodiment will not be described, and only differences from the first embodiment will be described.

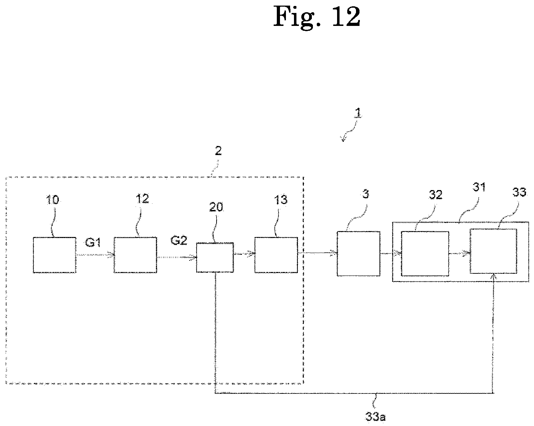

A difference of a device for producing an organic substance 1 in the fourth embodiment is that, as shown in Fig. 12, the device for producing an organic substance 1 includes the gasification furnace 10 that gasifies waste to generate a thermally decomposed gas, the reforming furnace 12 that reforms the thermally decomposed gas G1 discharged from the gasification furnace 10 and the heat exchanger 20 through which the thermally decomposed gas G1 that has passed through the reforming furnace 12 is passed to be cooled.

The device for producing an organic substance 1 (thermally decomposed gas purification/cooling device 2) includes the gasification furnace 10, the reforming furnace 12 and the heat exchanger 20, whereby it is possible to obtain the synthesis gas G2 having a high content rate of at least any of hydrogen and carbon monoxide and appropriately cooled with the heat exchanger 20.

In addition, in the above-described embodiment, a configuration in which the cyclone 11 is provided has been described; however, in the present embodiment, the cyclone 11 may not be provided. When the cyclone 11 is not provided, the synthesis gas G2 cooled in the heat exchanger 20 is supplied to the post-stage treatment device 13 without passing through the cyclone 11. For example, the cyclone 11 may not be provided in a case where a small amount of a solid impurity is contained in waste or in a case where the synthesis gas G2 is generated using a substance other than waste as a raw material.

The device for producing an organic substance 1 in the fourth embodiment is the same as the device for producing an organic substance 1 in the first or third embodiment except that the cyclone 11 is not provided. Therefore, the device for producing an organic substance 1 in the fourth embodiment includes the gasification furnace 10, the reforming furnace 12 and the heat exchanger 20, but the device for producing an organic substance 1 in the fourth embodiment may have, in addition to those members, the post-stage treatment device 13 or the like as appropriate. The post-stage treatment device 13 is as described in the first and third embodiments, and the device for producing an organic substance 1 preferably includes at least any of the gas cooling tower 21, the filtration-type dust collector 22 and the scrubber 23 in the post-stage treatment device 13.

At this time, the post-stage treatment device 13 can be configured as described in the first embodiment and the individual modification examples. That is, as shown in Fig. 2, the post-stage treatment device 13 can be configured to include the gas cooling tower 21 that is disposed in the post-stage of the heat exchanger 20, the filtration-type dust collector 22 that is disposed in the post-stage of the gas cooling tower 21 and the scrubber 23 that is disposed in the post-stage of the filtration-type dust collector 22. In addition, as shown in Fig. 3, the post-stage treatment device 13 can be configured to include the filtration-type dust collector 22 that is disposed in the post-stage of the heat exchanger 20 and the scrubber 23 that is disposed in the post-stage of the filtration-type dust collector 22. In addition, as shown in Fig. 4, the post-stage treatment device 13 can be configured to include the gas cooling tower 21 that is disposed in the post-stage of the heat exchanger 20 and the filtration-type dust collector 22 that is disposed in the post-stage of the gas cooling tower 21. In addition, as shown in Fig. 5, the post-stage treatment device 13 can be configured to include the gas cooling tower 21 that is disposed in the post-stage of the heat exchanger 20 and the scrubber 23 in the post-stage of the gas cooling tower 21. In addition, as shown in Fig. 6, the post-stage treatment device 13 can be configured to include the filtration-type dust collector 22 that is disposed in the post-stage of the heat exchanger 20.

In addition, the post-stage treatment device 13 may be configured as described in the third embodiment and the individual modification examples. That is, as shown in Fig. 8, the post-stage treatment device 13 may be configured such that the filtration-type dust collector 22 and the scrubber 23 are disposed in parallel in the post-stage of the heat exchanger 20 and the flow path-switching portion 41 is provided. Additionally, as described in the individual modification examples of the third embodiment, the post-stage treatment device 13 may be configured such that the differential pressure-measuring device 40 and the concentration-measuring device 42 are provided or may be configured such that the gas cooling tower 21 is provided in the pre-stage of the flow path-switching portion 41 and in the post-stage of the heat exchanger 20 (refer to Figs. 9 to 11). The details thereof are as described above and thus it will not be described.

### (Other embodiments)

For example, in the above description, a form in which one cyclone 11 is provided in the thermally decomposed gas purification/cooling device 2 has been described, but the number of the cyclones 11 is not limited to one, and two or more cyclones 11 may be provided. For example, the cyclones 11 may be provided in the post-stage of the gasification furnace 10 and in the post-stage of the reforming furnace 12, respectively, in the form. In addition, two or more cyclones 11 may be continuously provided.

Furthermore, in the above description, an aspect in which the synthesis gas G2 is obtained from waste in the gasification furnace 10 has been described, but the synthesis gas G2 may be generated from a substance other than waste in the gasification furnace 10. For example, the synthesis gas G2 may be generated from fossil resources such as natural gas, coal, heavy oil, petroleum discharged gas and oil shale, biomass other than waste or the like. In addition, the synthesis gas G2 may be a gaseous by-product in a variety of production processes such as a steel production process, and, for example, the gasification furnace 10 may configure a steel production facility or the like.

### Reference Signs List

1 Device for producing an organic substance
2 Thermally decomposed gas purification/cooling device
3 Organic substance generation portion
10 Gasification furnace
11 Cyclone
1 la Dust component supply path
12 Reforming furnace
13 Post-stage treatment device
19 Removal device
20 Heat exchanger
21 Gas cooling tower
22 Filtration-type dust collector
23 Scrubber
24 Water spray opening
25 Nozzle
26 Storage portion
27 Introduction path
28 Supply path
29 Discharge path
31 Separator
31a Water supply path
32 Solid-liquid separator
33 Distillation device
33a Heat energy path
40 Differential pressure-measuring device
41 Flow path-switching portion
42 Concentration-measuring device
G1 Thermally decomposed gas
G2 Synthesis gas

## Claims

1. A thermally decomposed gas purification/cooling device comprising:
a gasification furnace that gasifies waste to generate a thermally decomposed gas;
a cyclone through which the thermally decomposed gas discharged from the gasification furnace is passed to recover a dust component in the thermally decomposed gas; and
a heat exchanger through which the thermally decomposed gas that has passed through the cyclone is passed to be cooled.

2. The thermally decomposed gas purification/cooling device according to Claim 1,
wherein a temperature of the thermally decomposed gas to be supplied to the cyclone is 500°C or higher and 1,100°C or lower.

3. The thermally decomposed gas purification/cooling device according to Claim 1 or 2,
wherein the thermally decomposed gas is cooled to a temperature of 30°C or higher and 300°C or lower in the heat exchanger.

4. The thermally decomposed gas purification/cooling device according to any one of Claims 1 to 3, further comprising:
a reforming furnace that is disposed in a post-stage of the cyclone and reforms the thermally decomposed gas discharged from the gasification furnace.

5. The thermally decomposed gas purification/cooling device according to any one of Claims 1 to 3, further comprising:
a reforming furnace that is disposed in a pre-stage of the cyclone and reforms the thermally decomposed gas discharged from the gasification furnace.

6. The thermally decomposed gas purification/cooling device according to any one of Claims 1 to 5,
wherein the dust component recovered in the cyclone is supplied to the gasification furnace.

7. The thermally decomposed gas purification/cooling device according to any one of Claims 1 to 6, further comprising:
a gas cooling tower which is disposed in a post-stage of the heat exchanger and through which the thermally decomposed gas cooled in the heat exchanger is passed through to be cooled by water spray.

8. The thermally decomposed gas purification/cooling device according to any one of Claims 1 to 7, further comprising:
a filtration-type dust collector which is disposed in a post-stage of the heat exchanger and through which the thermally decomposed gas cooled in the heat exchanger is passed.

9. The thermally decomposed gas purification/cooling device according to any one of Claims 1 to 8, further comprising:
a scrubber which is disposed in a post-stage of the heat exchanger and through which the thermally decomposed gas cooled in the heat exchanger is passed.

10. The thermally decomposed gas purification/cooling device according to any one of Claims 1 to 9, further comprising:
a filtration-type dust collector and a scrubber,
wherein the filtration-type dust collector and the scrubber are disposed in parallel in a post-stage of the heat exchanger.

11. The thermally decomposed gas purification/cooling device according to Claim 10, further comprising:
a differential pressure-measuring device that measures a differential pressure between a pre-stage and a post-stage of the filtration-type dust collector.

12. The thermally decomposed gas purification/cooling device according to Claim 10 or 11, further comprising:
a concentration-measuring device that measures a concentration of at least any selected from a phase-transitable impurity and a solid impurity in the thermally decomposed gas discharged from the gasification furnace.

13. The thermally decomposed gas purification/cooling device according to any one of Claims 10 to 12, further comprising:
a flow path-switching portion that selectively switches a supply destination to the filtration-type dust collector or the scrubber through which the thermally decomposed gas is passed.

14. A device for producing an organic substance comprising:
an organic substance generation portion that generates an organic substance by bringing a synthesis gas obtained by treating a thermally decomposed gas with the thermally decomposed gas purification/cooling device according to any one of Claims 1 to 13 into contact with a microbial catalyst.

15. A thermally decomposed gas purification/cooling method comprising:
a step of gasifying waste with a gasification furnace to generate a thermally decomposed gas;
a step of passing the thermally decomposed gas discharged from the gasification furnace through a cyclone to recover a dust component in the thermally decomposed gas; and
a step of passing the thermally decomposed gas that has passed through the cyclone through a heat exchanger to cool the thermally decomposed gas.

16. The thermally decomposed gas purification/cooling method according to Claim 15,
wherein a temperature of the thermally decomposed gas to be supplied to the cyclone is 500°C or higher and 1,100°C or lower.

17. The thermally decomposed gas purification/cooling method according to Claim 15 or 16,
wherein the thermally decomposed gas is cooled to a temperature of 30°C or higher and 300°C or lower in the heat exchanger.

18. The thermally decomposed gas purification/cooling method according to any one of Claims 15 to 17, further comprising, in a post-stage where the thermally decomposed gas has passed through the cyclone:
a step of passing the thermally decomposed gas through a reforming furnace to reform the thermally decomposed gas discharged from the gasification furnace.

19. The thermally decomposed gas purification/cooling method according to any one of Claims 15 to 18, further comprising, in a pre-stage where the thermally decomposed gas is to be passed through the cyclone:
a step of passing the thermally decomposed gas through a reforming furnace to reform the thermally decomposed gas discharged from the gasification furnace.

20. The thermally decomposed gas purification/cooling method according to any one of Claims 15 to 19,
wherein the dust component recovered in the cyclone is supplied to the gasification furnace.

21. The thermally decomposed gas purification/cooling method according to any one of Claims 15 to 20, further comprising:
a step of passing the thermally decomposed gas cooled in the heat exchanger through a gas cooling tower to cool the thermally decomposed gas with water sprayed in the gas cooling tower.

22. The thermally decomposed gas purification/cooling method according to any one of Claims 15 to 21, further comprising:
a step of passing the thermally decomposed gas cooled in the heat exchanger through a filtration-type dust collector.

23. The thermally decomposed gas purification/cooling method according to any one of Claims 15 to 22, further comprising:
a step of passing the thermally decomposed gas cooled in the heat exchanger through a scrubber.

24. The thermally decomposed gas purification/cooling method according to any one of Claims 15 to 23, further comprising:
a step of passing the thermally decomposed gas through any of a filtration-type dust collector and a scrubber that are disposed in parallel in a post-stage of the heat exchanger.

25. The thermally decomposed gas purification/cooling method according to Claim 24, further comprising:
a step of measuring a differential pressure between a pre-stage and a post-stage of the filtration-type dust collector with a differential pressure-measuring device.

26. The thermally decomposed gas purification/cooling method according to Claim 24 or 25, further comprising:
a step of measuring a concentration of at least any selected from a phase-transitable impurity and a solid impurity in the thermally decomposed gas discharged from the gasification furnace with a concentration-measuring device.

27. The thermally decomposed gas purification/cooling method according to Claim 25 or 26, further comprising:
a step of selectively switching supply of the thermally decomposed gas to the filtration-type dust collector or the scrubber with a flow path-switching portion depending on a measurement result of at least any of the differential pressure-measuring device and the concentration-measuring device.

28. A method for producing an organic substance comprising:
a step of bringing a synthesis gas obtained by treating the thermally decomposed gas with a thermally decomposed gas purification/cooling method according to any one of Claims 15 to 27 into contact with a microbial catalyst to generate an organic substance.
